# EUROPEAN PATENT APPLICATION

(11) **EP 1 918 384 A1**
(43) Date of publication of application: **07.05.2008**
(21) Application number: 07123924.8
(22) Date of filing: 19.12.2003
(51) Int. Cl.: C12P 13/04

(54) **Method for the production of trehalose-free amino acids**

(30) Priority: 23.12.2002 DE 10261579
(62) Divisional of application: 03795933.5
(71) Applicant: BASF SE, 67056 Ludwigshafen (DE)
(72) Inventor: Klopprogge, Corinna, 68239, Mannheim (DE); Zelder, Oskar, 67346, Speyer (DE); Kröger, Burkhard, 67117, Limburgerhof (DE); Schröder, Hartwig, 69226, Nußloch (DE); Haefner, Stefan, 67346, Speyer (DE); Liebl, Wolfgang, 37120, Bovenden (DE)

(57) **Abstract**

The invention relates to a method for producing an amino acid comprising culturing a microorganism of the genus Corynebacterium or Brevibacterium wherein said microorganism is partially or completely deficient in at least one of the gene loci of the the group which is formed by otsAB, treZ and treS, and subsequent isolation of the amino acid from the culture medium.

## Description

### Summary

The analysis of the available C. glutamicum genome sequence data led to the proposal of the presence of all three known pathways for trehalose biosynthesis in bacteria, i.e. trehalose synthesis from UDP-glucose and glucose 6-phosphate (OtsA-OtsB pathway), from malto-oligosaccharides or α -1,4-glucans (TreY-TreZ pathway), or from maltose (TreS pathway). Inactivation of only one of the three pathways by chromosomal deletion did not have a severe impact on C. glutamicum growth while the simultaneous inactivation of the OtsA-OtsB and TreY-TreZ pathway or of all three pathways resulted in the inability of the corresponding mutants to synthesize trehalose and to grow efficiently on various sugar substrates in minimal media. This growth defect was largely reversed by the addition of trehalose to the culture broth.

In addition, a possible pathway for glycogen synthesis from ADP-glucose involving glycogen synthase (GlgA) was discovered. C. glutamicum was found to accumulate significant amounts of glycogen when grown under conditions of sugar excess. Insertional inactivation of the chromosomal glgA gene led to the failure of C. glutamicum cells to accumulate glycogen and to the abolishment of trehalose production in a Δ otsAB background, demonstrating that trehalose production via the TreY-TreZ pathway is dependent on a functional glycogen biosynthetic route.

The trehalose non-producing mutant with inactivated OtsA-OtsB and TreY-TreZ pathways displayed an altered cell wall lipid composition when grown in minimal broth in the absence of trehalose. Under these conditions, the mutant lacked both major trehalose-containing glycolipids, i.e. trehalose monocorynomycolate (TMCM) and trehalose dicorynomycolate (TDCM), in its cell wall lipid fraction. Our results suggest that a dramatically altered cell wall lipid bilayer of trehalose-less C. glutamicum mutants may be responsible for the observed growth deficiency of such strains in minimal media. The results of the genetic and physiological dissection of trehalose biosynthesis in C. glutamicum reported here may be of general relevance for the whole phylogenetic group of mycolic acid-containing coryneform bacteria.

### Introduction

Corynebacterium glutamicum is a Gram-positive soil bacterium that was originally isolated by its ability to produce and excrete glutamic acid (Kinoshita et al. 1957). Today, industrial amino acid production processes using genetically improved strains of this microorganism are used to satisfy the growing world market of amino acids, in particular L-glutamate and L-lysine .

In the classification system of bacteria, the genus Corynebacterium, together with mycobacteria, nocardia, rhodococci and some related taxa, belongs the group of mycolic acid containing actinomycetes. These genera are also phylogenetically related. Unusual for Gram-positive bacteria, their cell walls contain a characteristic hydrophobic layer outside of the plasma membrane. It was shown that this layer plays an important role in the drug and substrate permeability in coryneform bacteria. In contrast to the Gram-negative bacteria where the outer membrane is composed of phospholipids and lipopolysaccharides, the predominant constituents of the outer lipid layer of corynebacteria and related taxa are the mycolic acid esters. Recently it was shown that the outer hydrophobic barrier of corynebacterial cells represents a lipid bilayer composed of both covalently cell-wall-linked mycolates and non-covalently bound glycolipids Two trehalose-containing corynomycolic acid esters, i. e. trehalose monocorynomycolate (TMCM) and trehalose dicorynomycolate (TDCM) were shown to be the major free lipid fractions of this lipid bilayer The presence of trehalose in C. glutamicum is not restricted only to these two structural components. Significant amounts of free trehalose are observed in C. glutamicum cells as a response to hyperosmotic stress. In addition, trehalose was found as one of the by-products excreted into the growth medium during fermentation of the lysine-overproducing C. glutamicum strain ATCC 21253.

Trehalose (-D-glucopyranosyl -D-glucopyranoside), a non-reducing disaccharide widely spread in nature, has been found in a large variety of both pro- and eukaryotic organisms, ranging from bacteria to plants, insects and mammals. The biological role of trehalose varies significantly in different organisms. While in bacteria it can be used as a carbon source (E. coli, B. subtilis), or is synthesized as a compatible solute under osmotic shock conditions (E. coli), or plays a structural role (Corynebacteriaceae). In yeast and filamentous fungi trehalose is stored intracellularly primarily as a reserve carbohydrate or as a protector against different stress factors. In several species of insects, trehalose is accumulated for use as a rapidly utilizable sugar source during the flight.

Several possible pathways for trehalose biosynthesis were observed in different organisms. The most abundant pathway, i.e. trehalose synthesis from UDP-glucose and glucose 6-phosphate (OtsA-OtsB pathway; ), is widely represented in the prokaryotes and the only one known in the eukaryotes. The first step of this pathway is the condensation of glucose 6-phosphate with UDP-glucose resulting in the formation of trehalose 6-phosphate and release of UDP. Trehalose is then formed by dephosphorylation of trehalose 6-phosphate. This biosynthetic reaction mechanism was found in bacteria like E. coli and yeast. In E. coli, the reactions are catalyzed by the enzymes trehalose 6-phosphat synthase (OtsA) and trehalose 6-phosphat phosphatase (OtsB). The transcription of both enzymes is induced by osmotic shock or upon entry into the stationary growth phase. In S. cerevisiae, both reactions are catalyzed by an enzyme complex which consists of two catalytic polypeptides, TPS1 and TPS2, and one regulatory subunit responsible for activation of the complex under stress conditions. Coding regions for corresponding enzymes were identified also in the genomes of higher eukaryotes. An alternative pathway for trehalose synthesis that uses glycogen as the initial substrate (TreY-TreZ pathway; ) was discovered in some bacteria and archaea. In this case, first the terminal (1→4) glycosidic bond at the reducing end of the -glucan polymer is transformed into an (1→1) glycosidic bond via transglycosylation, resulting in the formation of a terminal trehalosyl unit. Subsequently, trehalose is released from the polymer' s end via hydrolysis. The enzymes involved in this pathway are maltooligosyltrehalose synthase (TreY) and maltooligosyltrehalose hydrolase (TreZ). An additional pathway for trehalose synthesis, which is based on trehalose production from maltose, was discovered in some bacteria. In this case, trehalose is synthesized by a single reaction catalyzed by trehalose synthase (TreS), which converts the (1→4) glycosidic bond of maltose into an (1→1) bond to form trehalose (TreS pathway; ). It was shown that, although close in their intramolecular transglycosylation activity, TreY and TreS can not substitute each other in vivo because of the differences in their substrate specificities.

In most bacteria studied only one of the three biosynthesis pathways was found, with the exception of Mycobacterium species. Strains of this genus have been shown by in vitro assays to possess all three pathways for trehalose synthesis. The question arises as to what biological role trehalose has in these bacteria that makes necessary a threefold coverage of its biosynthesis. Also, it is of interest to analyze if Corynebacterium, which is phylogenetically related to Mycobacterium, contains a similarly rich outfit of trehalose biosynthesic pathways.

To answer these questions we have scoured the available genome data in order to identify the pathways used for trehalose biosynthesis in C. glutamicum. By inactivation of chromosomal genes coding for enzymes of the identified pathways we intended to probe the role of the different pathways in the in vivo synthesis of trehalose. Also, by inactivation of these genes we intended to reduce or even abolish trehalose synthesis in order to reveal the physiological role of this sugar in C. glutamicum.

The invention provides methods for producing an amino acid, preferably of the group consisting of lysine, threonine, methionine, and glutamate, comprising culturing a microorganism of the genus Corynebacterium or Brevibacterium wherein said microorganism is partially or completely deficient in at least one of the gene loci of the group which is formed by otsAB, treZ and treS, and subsequent isolation of the amino acid from the culture medium.

Preferred embodiments of the invention are methods for producing an amino acid comprising culturing a microorganism of the genus Corynebacterium or Brevibacterium wherein said microorganism is partially or completely deficient in the gene loci of otsAB alone or in combination with the gene loci of glgA or glgA and treS.

Another preferred embodiment of the invention are methods for producing an amino acid comprising culturing a microorganism wherein said microorganism is deficient in the gene loci of otsAB in combination with treZ alone or in combination of treZ and treS.

The gene loci have the following meaning:
glgA: glycogen synthase
otsA: trehalose 6-phosphat synthase
otsB: trehalose 6-phosphat phosphatase
treS: trehalose synthase
treY: maltooligosyltrehalose synthase
treZ: maltooligosyltrehalose hydrolase
otsAB stands for either otsA or otsB or otsA and otsB.

The gene sequences of the coding parts of the above-mentioned gene loci are known in the art e.g. from WO 2001/00843 otsA (SEQ ID NO:17; otsB (SEQ ID NO: 1139; treZ (SEQ ID NO: 1145) or from WO 2002/51231 treS (SEQ ID NO:3) or from EP 1108790 glgA (SEQ ID NO: 1238):

According to the invention a microorganism of the genus Corynebacterium or Brevibacterium which is able to produce an amino acid if it is cultured unde suitable conditions is modulated in specific genes involved in trehalose metabolism in order to prevent the synthesis of trehalose in said microorganism. The modulation of the microorganism is performed in such a way that the resulting modulated microorganism is deficient in at least one of the gene loci of the group which is formed by otsAB, treZ and treS. The deficiency can be partially or completely.

Partially deficient means the a part of the gene locus has been changed by inserting, deleting or substituting or or more nucleotides of this gene locus. Deficient means that the normal function of that gene locus has been changed. A partially deficient microorganism with respect to a specific gene locus means that the respective gene locus retains some of its original function whereas a completely deficient microorganism means that the respective gene locus has completely lost its original function.

A preferred method of producing microorganisms deficient in a specific gene locus is to delete one or more nucleotides of said locus up to the complete deletion of the whole gene locus.The deletion can be made in the coding region or in the regulatory region, e.g. in the promotor region, of the respective gene locus.

The microorganims according to the invention have a reduced (up to 0%) capacity to produce trehalose. As a consequence the productivity of this microorganisms with respect to amino acids is improved.

### Materials and Methods

### Strains, media and cultivation

The C. glutamicum strains and plasmids which were used in this study are listed in Table.1. Additionally, the E. coli strains XL1-blue (Bullock et al., 1987) and S17-1 (Simon et al., 1983) were used for plasmid construction and mobilization of integration vectors in to C. glutamicum, respectively. The restriction deficient C. glutamicum strain R163 (Liebl et al., 1989a) was used for preparation of plasmid constructs preliminary to their electroporation in the C. glutamicum type strain. The strains were maintained on LB plates with an antibiotics supplementation by requirement.

For investigation of trehalose synthesis, C. glutamicum strains were grown on defined BMC-media (Liebl et al., 1989b) supplemented with different amounts of sucrose or other carbon sources as mentioned in the text. Cells inoculated from LB plates in 5 ml LB and grown overnight (30°C; 210 rpm) were used as precultures for the inoculation of tubes with 5 ml or flasks with 30 ml BMC broth. The inoculation density of the main cultures was OD₆₀₀ 0.1-0.2. When required, kanamycin was added to the media at a final concentration of 20 µg ml⁻¹. All cultures were grown on a rotary shaker (30°C; 210 rpm). Rapid shaking of more than 200 rpm was found to be important for growth of trehalose-non-producing mutants (see text). Samples were taken after different periods of incubation. The growth of cultures was monitored by OD measurements at 600 nm using an Ultrospec 3000 spectrophotometer (Pharmacia, Uppsala, Sweden). If necessary the samples were diluted to an OD lower than 0.3 prior to the measurements.

### Recombinant DNA techniques

Basic methods such as plasmid isolation, DNA restriction and ligation were performed according to Sambrook et al. (1989). Restriction endonucleases and DNA modification enzymes were purchased from MBI Fermentas (St. Leon-Rot, Germany) or New England Biolabs (Frankfurt, Germany). C. glutamicum plasmid DNA was isolated using the alkaline extraction procedure (Birnboim & Doly, 1979) after preliminary treatment of the cells with 10 µg ml⁻¹ lysozyme for 30 min at 37°C. Genomic DNA from C. glutamicum was isolated as described by Lewington et al. (1987). PCR reactions were carried out using Pfu polymerase (Promega, Mannheim, Germany). Some of the PCR products were cloned directly into the vector pCR4 using the TOPO^{R} Cloning Kit (Invitrogen, Karlsruhe, Germany) according to the manufacturer' s instructions.

### Construction of Δ otsAB, Δ treZ , Δ treS and glgA::Km mutants of C. glutamicum DSM20300

The two-step recombination system (Schaefer et al., 1994), based on the inability of C. glutamicum carrying the sacB gene to grow in media with high sucrose concentrations, was used for the chromosomal inactivation of the trehalose biosynthesis genes of C. glutamicum. For each planned inactivation experiment, a mobilizable C. glutamicum integration vector was constructed which contained the gene of interest but with an internal deletion, thus providing two homology regions for recombination.

For inactivation of the otsA-otsB genes two chromosomal DNA regions were amplified separately and re-ligated resulting in the in-frame deletion of both genes. A fragment of 1.5 kb carrying the entire otsA ORF was amplified using the primers tre351_f and tre351_r (Table 2) and cloned into the EcoRV restriction site of pBluescript KS, resulting in pBlueKS::otsA. Then, a 0.65 kb region carrying part of otsB was amplified with the primers otsAB_f and otsAB_r. The PCR product, cut with HindIII and SphI, served to replace a 0.90 kb HindIII-SphI fragment of the otsA-carrying plasmid, resulting in the in-frame fusion of the 5' -part of otsA with the 3' -part of otsB genes. Using Xbal, the resulting Δ otsAB ORF was cloned into the mobilizable integration vector pCLiK8.2 for inactivation of the C.glutamicum chromosomal otsAB locus.

A mobilizable treZ inactivation plasmid was constructed as follows: a 2.5 kb treZ fragment was amplified with the primers treZ_f and treZ_r. The PCR product was cut with Xbal and cloned into pCLiK3, before introduction of an internal 0.65 kb in-frame deletion into treZ with SaII. The Δ treZ gene was cloned via Xbal into the mobilizable integration vector pCLiK8.2.

For chromosomal inactivation of treS, the gene cloned in pBluescriptKS after amplification with the PCR primers treS_f and treS_r. Upon digestion of the resulting plasmid with EcoRV and Styl, and treatment with Klenow enzyme the plasmid was religated, resulting in an 0.65 kb in-frame deletion in the cloned treS ORF. The truncated gene was cloned into the mobilizable plasmid pK18mobsac (Schaefer et al., 1994) using Xbal.

The three final constructs for inactivation of the OtsA-OtsB, TreY-TreZ and TreS pathways, designated pCLiK8.2::Δ otsAB, pCLiK8.2::Δ treZ and pK18ms::Δ treS, respectively, were transformed into the strain E.coli S17-1 and mobilized into heat-stressed C. glutamicum according to the procedure described by Schäfer et al. (1990). Successful first recombinants (chromosomal integration mutants) were selected by plating on LB plates containing kanamycin at 20 µg ml⁻¹. For selection of the second recombination event, the integration mutants were plated on agar plates containing 5-10 % (w/v) sucrose. In some cases (see Results), trehalose was added at 2 % (w/v).

A putative glycogen synthase gene (glgA) was inactivated by single-step chromosomal integration. For this purpose, a 0.6 kb internal fragment of glgA was amplified using glg_f and glg_r as the PCR primers. The PCR product was cloned into the integration vector pCLiK6 using its unique Xbal site. The resulting plasmid was mobilized using E. coli S17-1 as described above. The integration mutants were selected on LB media supplemented with kanamycin.

The genotype of the obtained mutants was verified by Southern blot analysis and with specific PCR reactions.

### Construction of pWLQ2 otsAB, pWLQ2 otsA, pWLQ2 treZ , and pWLQ2 treS

Expression plasmids carrying the various trehalose biosynthesis genes were constructed using the C. glutamicum-E. coli shuttle expression vector pWLQ2 (Liebl et al., 1992). The plasmid pBlueKS::otsA in which otsA gene was initially cloned after PCR amplification as described above was used for the construction of an expression plasmid carrying the otsA gene. A 1.6 kb BamHI-Sall fragment of pBlueKS::otsA carrying the otsA gene was ligated with pWLQ2 opened with the same enzymes. In the resulting plasmid (pWLQ2 otsA) the otsA gene is under the control of P_{tac} promoter. For construction of pWLQ2 otsAB, the otsB gene was amplified from the C. glutamicum chromosome using the primers otsB_f and otsB_r. After cloning the PCR product in pCR4-TOPO, the 1 kb BamHI fragment was excised and inserted into the BamHI site of pWLQ2::otsA. In the resulting plasmid, designated pWLQ2::otsAB, both ots genes are co-expressed under regulation of the P_{tac} promoter.

For construction of pWLQ2 treZ, a 2.5 kb PCR product generated with the primers treZ_f2 and treZ_r2 was cloned into pCR4-TOPO. Then, the treZ gene was excised with BamHI and recloned in the BamHI site of pWLQ2. The plasmids obtained were checked via restriction analysis for the correct orientation of treZ with respect to the P_{tac} promoter. For the construction of pWLQ2 treS, the chromosomal C. glutamicum treS gene was amplified as a 2 kb fragment using the primers treS_f3 and treS_r3. After initial cloning into pCR4-TOPO, the treS gene was excised and recloned into pWLQ2 using artificially added Sail sites. The plasmid pWLQ2::treS was isolated in which treS is orientated colinearily to the P_{tac} promoter. All plasmids were transformed into C. glutamicum strains by electroporation (Liebl et al., 1989a), normally after passaging them through a restriction-deficient strain to increase the efficiency. The strains were grown with kanamycin selection at 20 µg ml⁻¹. Promotor P_{tac}-driven gene expression was induced by addition of IPTG at a final concentration of 1 mM.

### Isolation and analysis of lipids

Cell lipids were isolated as described by Puech et al. (2000). The cells were harvested and washed after approximately 10h of incubation (growth at 210 rpm at 30°C) as described above (see sample preparation). For lipid extraction the wet cells were suspended in CHCl₃/CH₃OH [1:1 (v/v)] and shaked at room temperature for 16h. Remaining bacterial residues were re-extracted twice with CHCl₃/CH₃OH [2:1 (v/v)] and the organic phases were pooled and concentrated in a vacuum cetrifuge. Watersoluble contaminants were removed by additional extraction with water [2:1 (v/v)] and the organic phases were freeze-dried, yielding the crude lipid extracts. Lipid extracts were dissolved in chloroform at a final concentration of 50 µg µl⁻¹ and analyzed by TLC analysis. Samples were applied to silica gel-coated aluminum plates (type G-60, 5 x 10 cm, Merck) and developed with CHCl₃/CH₃OH/H₂O [30:8/1 (v/v)] in a tightly sealed chamber at 4 °C. Glycolipids were visualized by spraying with an 0.2 % (w/v) anthrone solution in H₂SO₄ conc. followed by heating (at 100°C for 10-15 min).

Quantification of the trehalose content of the lipid extracts was made after saponification of the crude lipid extract according to Liu & Nikaido (1999), with modifications: aliquots of the samples were taken before the water extraction, freeze-dried and dissolved in 5 % (w/v) potassium hydroxide. The samples were incubated for 1 h at 100°C, cooled, and aliquots were directly used for trehalose determination by high-pH HPLC (see below).

### Sample preparation for trehalose and glycogen determination

Samples of cultures (1.5 ml) were rapidly cooled on ice and centrifuged (13,000 rpm, 4°C, 15 min). All subsequent manipulations were done at 4°C. The supernatant was collected and frozen at - 20°C for subsequent extracellular trehalose determination. The cells were washed with BMC medium and also stored as a pellet at -20°C. In order to minimize changes in the extracellular osmotic conditions, ice-cold media with the same salt and sugar composition as the growth media was used for washing. Aliquots of the washed cells were used for determination of cell dry weight.

Cells were opened by sonication (40 % amplitude, 0.5 sec cycle) in 500 µl 10 mM sodium/potassium phosphate buffer pH 6. Cellular debris was removed by centrifugation (13,000 rpm, 4°C, 15 min) and the supernatant was used for trehalose and/or glycogen determination.

### Trehalose Determination

An enzymatic trehalose determination assay was used which was based on the quantitative enzymatic hydrolysis of trehalose to two molecules of glucose, using recombinant trehalase from E. coli. For this purpose, the E.coli trehalase TreA was overexpressed and partially purified as described by De Smet et al. (2000). Glucose was then determined by a oxidase/peroxidase method. Samples of 5 to 20 µl were incubated with or without recombinant trehalase (5 U) in 90 ml of 10 mM sodium/potassium phosphate buffer pH 6.0 for 1 h at 37 °C. The glucose liberated was assayed by the addition of 900 µl freshly prepared enzyme-color reagent solution from a commercially available glucose detection Kit (Sigma 510-DA). After 30 min of incubation at 37°C glucose was measured spectrophotometrically at λ = 450 nm. Trehalose was calculated from the difference of the glucose amounts in the samples with and without trehalase treatment. A significant background was observed during the measurement of extracellular trehalose at a high concentration of maltose, i.e. in culture supernatants of 10 % (w/v) maltose-containing BMC broth, which is caused either by contamination of the maltose with trehalose or by non-specific intereference of maltose with the enzymatic trehalose assay. The background was determined by the enzymatic assay of samples of sterile maltose BMC and subtracted from the values obtained from culture supernatants.

For more complex samples such as crude cell extracts where a high background of glucose was observed, a chromatographic method for trehalose determination was used. In this case, trehalose was measured with high-pH ion chromatography (HPIC) at room temperature using a Carbo-Pak PA1 column installed in a DX500-HPLC system (DIONEX) supplied with a pulsed amperometric detector ED40. Samples of 25 µl of 10-fold diluted crude extracts were applied to the column. Elution was made with a linear gradient from 0 to 80 mM sodium acetate in a 150 mM sodium hydroxide solution. The column was regenerated by a 10 min wash with 500 mM sodium acetate followed by 10 min equilibration with 150 mM sodium hydroxide. Trehalose was detected as a single peak with a retention time of approximately 3.3 min. Trehalose quantification was based on calibration with defined amounts of a trehalose standard solution.

### Glycogen Determination

The amount of intracellular glycogen in C. glutamicum was assayed by hydrolysis with amyloglucosidase. For this purpose, samples (200 µl) of crude cell extracts (prepared as described above) were mixed with 2 volumes of 97 % (v/v) ethanol, pelleted and redissolved with heating in the same volume of 10 mM sodium/potassium phosphate buffer pH 6.0. Samples of 5 to 50 µl were incubated with amyloglucosidase (60 mU; Boehringer Mannheim) in 90 ml 100 mM sodium acetate buffer pH 4.5 for 1 h at 37°C. The amount of glucose liberated was determined enzymatically as described above. The amount of glycogen was calculated from the difference in glucose concentration between the amyloglucosidase-treated samples and control samples without amyloglucosidase.

### Results

### Analysis of C. glutamicum genome sequence data

The available sequences from the raw C. glutamicum genome data (www.ncbi.nlm.nih.gov/PMGifs/Genomes/micr.html; accession no. NC_003450) were screened for the presence of ORFs with similarity to genes known to be involved in trehalose metabolism (summarized in Table 3). For the initial identification of potential candidates the suggested genome annotations were used. In addition, a BLAST search was made that was based on the enzymes for trehalose synthesis of Mycobacterium tuberculosis, a human pathogen phylogenetically related with Corynebacterium bacteria, which possesses all three known pathways for trehalose biosynthesis (De Smet et al., 2000). ORFs with high similarity to all 5 genes involved in the different pathways were also found in C. glutamicum .

The ORFs Cgl2573 and Cgl2575 were designated as otsA and otsB, respectively, because they putatively encode polypeptides with significant similarity to the enzymes trehalose 6-phosphat synthase and trehalose 6-phosphat phosphatase of the OtsA-OtsB pathway. Both genes are separated by an additional ORF (Cgl2574) with the same orientation as otsA and otsB . In addition, two identically orientated ORFs (Cgl2571, Cgl2572) are present upstream of otsA. Recently it was shown that one of them (Cgl2571) encodes a transmembrane threonine exporter (Simic et al., 2001). The translation products of the ORFs Cgl2572 and Cgl2574 do not share significant similarity with other proteins, thus their physiological role in C. glutamicum is unknown at present. However, their close neighborhood to the ots genes and their collinear orientation to these genes suggests that they may be co-transcribed with and may play a physiological role connected to otsA and otsB. Finally, an oppositely oriented ORF was found downstream of otsB. Its predicted amino acid sequence revealed a high degree of similarity to the Lacl-family of transcription regulators. It is not known whether this ORF is involved in the regulation of the otsA and otsB genes.

A BLAST search of the C. glutamicum genome with the sequences of the M. tuberculosis trehalose biosynthesis enzymes revealed two ORFs, Cgl2075 and Cgl2066, that showed significant similarity to the TreY and TreZ enzymes, respectively, which are involved in trehalose synthesis from glycogen. Their chromosomal organization in C. glutamicum differs significantly from that of similar genes in other organisms where both genes are clustered together, often even overlapping each other (Maruta et al., 1996a-c; Cole et al., 1998). Although localized in the same region of the C. glutamicum chromosome, the treY and treZ genes of this organism are separated by a stretch of more than 8kb length which contains seven ORFs. Based on the annotations available and own sequence comparisons, a physiological connection cannot be proposed between treY and treZ genes and the ORFs in between. In Sulfolobus acidocaldarius, M. tuberculosis and Arthrobacter sp. Q36 the treY and treZ genes constitute an operon with a third gene designated as treX, which is thought to have a glycogen debranching function in the trehalose biosynthesis process (Maruta et al., 1996c; Maruta et al., 2000; Cole et al., 1998). A BLAST search of the C. glutamicum genome with the sequence deduced from Arthrobacter sp. treX revealed an ORF (Cgl2054) with similarity to the glycogen debranching enzymes of different bacteria localized 10 kb upstream of treY gene (data not shown). The fact that treY, treZ and Cgl2054 all have the same orientation on the C. glutamicum genome and are separated from each other merely by several kb may indicate that this distribution is the result of intragenomic rearrangements of originally clustered genes.

Also, an ORF (Cgl2250) was identified in the C. glutamicum genome which is significantly related to the trehalose synthase genes of other bacteria (Table3). This gene was designated treS. The start of the open reading frame located immediately downstream of treS (Cgl2251;) overlaps the 3' - end of the treS ORF by 4 bp. ORFs with high similarity to Cgl2251 are found also directly downstream of treS in Streptomyces coelicolor and M. tuberculosis. In other bacteria like Ralstonia solanacearum, Pseudomonas aeruginosa and Chlorobium tepidum, the treS and Cgl2251 homologues are fused in one ORF. Although nothing is known about the properties and physiological role of these putative Cgl2251-similar proteins, the genome data suggest a close functional connection with trehalose synthase.

To check the possibility for glycogen to serve as a substrate for trehalose biosynthesis via the TreY-TreZ pathway, the C. glutamicum genome was scoured for putative genes for enzymes that may be involved in glycogen synthesis (Preiss & Greenberg, 1964). Two ORFs, Cg11073 and Cg11072, were found whose translation products are highly similar to the (putative) enzymes ADP-glucose pyrophosphorylase (GlgC) and glycogen synthase (GlgA) (Table3). Both ORFs are situated next to each other but are oriented divergently, with their start codons separated by 51 bp . An additional ORF, Cgl1071, which is situated directly downstream of the glgA gene, is similar to known β -fructosidases and levanases. An additional ORF (Cgl0401) with significant similarity to (putative) glycogen synthase enzymes was found (Table3). However, due to the genetic surroundings of Cgl1072 this gene and not Cgl0401 was preferred for investigation of its role in glycogen synthesis.

In summary, exploration of the C. glutamicum genome data indicated the presence of all three pathways for trehalose biosynthesis observed in bacteria, thus suggesting a similar gene outfit for this purpose as in the related M. tuberculosis. In addition, the genome data suggested the presence of the pathway for glycogen synthesis in C. glutamicum. A series of experiments was designed in order to probe the role of the multiple trehalose synthesis pathways for growth of this organism and to elucidate the possible interconnection of glycogen synthesis and trehalose production in C. glutamicum.

### Accumulation of free trehalose by C. glutamicum

Lysine-overproducing mutants of C. glutamicum accumulate up to 6 g/l trehalose in the culture broth under conditions close to those used for industrial lysine production. Attempts to connect this significant trehalose accumulation with changes in the osmolarity of the growth medium, using the type strain of C. glutamicum and NaCl addition to increase the osmolarity, were not successful. On the other hand, when sucrose was used instead of NaCl for adjustment of the medium' s osmolarity, a significant longterm increase of the extracellular trehalose was observed.

The growth and trehalose accumulation by the type strain of C. glutamicum in minimal BMC medium with two different sugar concentrations, i.e. 0.5 % (w/v) sucrose and 10 % (w/v) sucrose), was followed. In the case of the low sugar medium C. glutamicum stopped its growth at an OD₆₀₀ of about 12, due to substrate limitation. In this case the trehalose accumulated in the culture broth did not exceed 0.1 g/l. In contrast, when grown with an excess of sucrose the bacteria reached a final OD₆₀₀ of more than 16. Under these conditions, the type strain accumulated up to 0.9 g/l trehalose during the late logarithmic and the stationary phase. Monitoring of the intracellular trehalose level showed that in the case of high sucrose supply, intracellular levels of about 20 µg trehalose per mg dry cell weight were reached, which is about four times the maximum intracellular trehalose level detected in the case of low sucrose supplementation. Under low- as well as high-sucrose conditions, the intracellular trehalose concentration dropped to extremely low values in stationary-phase cells.

The fact that the extracellular trehalose accumulation correlated with sugar excess in the media, in concert with the knowledge of the presence of putative genes for trehalose production from glycogen or other glucopolysaccharides in the C. glutamicum genome, prompted us to check for the presence of glycogen as a possible substrate for trehalose production in the cells. Indeed, using the method described by Brana et al. (1982), which is based on the determination of glycogen as glucose after amyloglucosidase hydrolysis, it was shown that C. glutamicum is able to produce glycogen when supplied with a surplus of sucrose. Under conditions of excess sucrose, glycogen accumulation was found to correlate with trehalose accumulation (Fig 3).

### Inactivation of the C. glutamicum trehalose biosynthesis pathways

In order to determine the role of the different pathways proposed from the genome data analysis in C. glutamicum trehalose biosynthesis in vivo, three mutants were constructed by chromosomal inactivation of at least one gene of each pathway. Specific mobilizable gene inactivation vectors were constructed for each of the chromosomal loci of interest and used for the introduction of deletions in the chromosome of C. glutamicum DSM20300 by a two-step homologous recombination-dependent gene exchange strategy as described in Materials and Methods. For inactivation of the OtsA-OtsB pathway a 2.4 kb chromosomal fragment was removed, resulting in the in-frame fusion of truncated otsA and otsB genes. In this mutant, designated C. glutamicum Δ otsAB, more than 70 % of the otsA gene, the entire ORF Cgl2574, and more than 95 % of otsB were deleted . Inactivation of the TreY-TreZ pathway was achieved by in-frame deletion of a 645 bp fragment of the treZ gene. Preceding efforts to inactivate the first gene of the pathway (treY) were abandoned after unsuccessful attempts, perhaps due to polar effects of such deletions on the Cgl2067 open reading frame. The third proposed pathway for trehalose synthesis in C. glutamicum, i. e. the TreS pathway that uses maltose as a precursor, was inactivated by the in-frame deletion of a 459 bp internal fragment of treS, the only gene directly involved in this biosynthesis route. By comparing the in vitro trehalose synthase activity of the intact and the truncated enzymes obtained by heterologous expression in E.coli it was possible in this case to confirm that the truncated gene no longer encoded a functional trehalose synthase enzyme (data not shown) before replacement of the chromosomal treS gene. Thus, three C. glutamicum DSM20300 single mutants were obtained and named Δ otsAB, Δ treZ and Δ treS, according to the pathway targeted for inactivation in each case.

Based on the single mutants just described, all possible combinations of double mutants (Δ otsAB/Δ treZ, Δ otsAB/Δ treS, Δ treZ/Δ treS) as well as the triple mutant (Δ otsAB/Δ treZ/Δ treS) inactivated in all three trehalose synthesis pathways were constructed. During the construction of the Δ otsAB/Δ treZ and the Δ otsAB/Δ treZ/Δ treS mutants we faced difficulties to obtain the second-step (vector excision) recombinants carrying the desired deletion. Instead of obtaining nearly equal numbers of the desired deletion variants and clones resulting from reversion of the vector integration event (Schäfer et al., 1994), only the latter type of second-step recombinants were obtained. The problem was overcome after addition of 2 % (w/v) trehalose to the medium used for the sacB-based selection of clones carrying the second recombination event. This interesting observation was a first indication that these two mutant strains had severe difficulties to grow without trehalose in the medium.

Attempts to grow either one of the Δ otsAB/Δ treZ and Δ otsAB/Δ treZ/Δ treS mutant strains in liquid minimal media without trehalose with moderate shaking (about 150 rpm) showed that both mutants were unable to grow properly under these conditions. After 2-3 hours of incubation these mutants produced aggregates of cells which rapidly sedimented at the bottom of the culture tubes , which probably leads to oxygen- and nutrient-limiting conditions and impairs further growth. Although the increase of culture agitation to 210 rpm resulted in the improvement of growth, the strains carrying mutations in both the OtsA-OtsB and the TreY-TreZ pathways were significantly impaired in their ability to grow in minimal media in comparison to the other trehalose synthesis mutants and the type strain.

Experiments to measure the intra- and extracellular accumulation of trehalose by the C. glutamicum type strain and the mutants were made in tubes with 5 ml of 10 % (w/v) sucrose-containing BMC media. A more than 50 % decrease of the intracellular trehalose concentration was observed in the mutants carrying either the Δ otsAB or the Δ treZ mutation, and the complete absence of intracellular trehalose was noted in the strains simultaneously carrying both mutations. Also, in comparison with the wild-type strain, the Δ otsAB, Δ treZ, Δ otsAB/Δ treS and Δ treZ/Δ treS mutants exerted a significant (about 20 to 50 %) decrease in the levels of extracellular trehalose accumulation. In the double mutant Δ otsAB/Δ treZ and the triple mutant Δ otsAB/Δ treZ/Δ treS no significant amount of extracellular trehalose was detected. In contrast, the mutant inactivated only in the TreS pathway showed only a slight decrease in the intracellular and almost no change in the extracellular trehalose levels when compared to the type strain.

The mutants impaired in growth on sucrose-containing minimal media, i. e. A otsAB/Δ treZ and Δ otsAB/Δ treZ/Δ treS, were checked for their ability to grow on different substrates known to be utilized by C. glutamicum (Table 4). For this purpose, the cells were grown in tubes containing 5 ml BMC media supplemented with different carbon sources at a final concentration of 1 % (w/v). Cultivation was carried out at 30°C at 150 rpm. It is noteworthy in this context that C. glutamicum DSM20300 is unable to grow on trehalose as the sole source of carbon and energy. On most of the sugar substrates tested the wild-type strain reached a maximum optical density of above 15, while the mutant strains displayed significantly impaired growth. In contrast, growth of the mutants on acetate or pyruvate was not as severely affected as growth on sugar substrates. In sucrose cultures supplemented with trehalose both mutants showed merely a slight decrease in their growth when compared with the wild-type strain. The phenomenon of complementation of the mutants by trehalose addition was investigated in more detail by recording growth curves.

### Complementation of Δ otsAB/Δ treZ and Δ otsAB/Δ treZ/Δ treS by addition of trehalose

The mutants Δ otsAB/Δ treZ and Δ otsAB/Δ treZ/Δ treS were significantly impaired in their ability to grow in minimal BMC media while their growth rates did not differ significantly from that of the type strain when grown on complex LB media (not shown). In search for the component or components needed for normal growth of the mutants, which are obviously present in LB media but absent in minimal media, we attempted to supplement the BMC minimal medium with different low molecular weight components such as the osmo-protectants L-proline, betain and also trehalose. Addition of proline and betain (at 20 mM) did not improve the mutants' growth (data not shown) while the addition of 2 % (w/v) trehalose to the BMC media resulted in nearly the same growth rate and final culture density as the wild-type control. These data demonstrate that the simultaneous inactivation of both the OtsA-OtsB and the TreY-TreZ pathways leads to trehalose auxotrophy of C. glutamicum.

### Growth of Δ otsAB/Δ treZ and Δ otsAB/Δ treZ/Δ treS on maltose

The fact that the double mutant Δ otsAB/Δ treZ and the triple mutant Δ otsAB/Δ treZ/Δ treS displayed similar growth behaviour in minimal media with most of the substrates tested (Table 4) indicates that the presence of an intact treS gene had no significant effect on growth under these conditions. Taking into account that trehalose synthase (TreS) catalyses trehalose production from maltose we investigated the growth phenotype of both mutants on BMC minimal media supplemented with 1 % (w/v) maltose as the sole carbon source (Table 4;). While growth of the triple mutant Δ otsAB/Δ treZ/Δ treS was significantly impaired in this medium, the Δ otsAB/Δ treZ strain in which the treS gene is still intact displayed a growth rate comparable with the wild-type strain.

In addition, the intra- and extracellular accumulation of trehalose by both mutants and the type stain grown at high maltose concentrations was checked. Under these conditions, interestingly, the intracellular concentration of trehalose measured in the mutant Δ otsAB/Δ treZ was similar to the concentration found in the type strain, while the triple mutant Δ otsAB/Δ treZ/Δ treS was devoid of intracellular trehalose. This result, in concert with the differences observed between the Δ otsAB/Δ treZ and Δ otsAB/Δ treZ/Δ treS mutants grown on maltose in comparison to growth on the other substrates (Table 4), suggests that the TreS pathway is functional and able to supply sufficient amounts of trehalose for C. glutamicum growth only in the presence of maltose. It is noteworthy that there was no significant accumulation of extracellular trehalose by both mutants, indicating that in the type strain the OtsA-OtsB and/or TreY-TreZ pathways are responsible for the extracellular appearance of trehalose.

### Plasmid complementation of Δ otsAB mutations

Expression plasmids carrying the otsA gene (pWLQ2::otsA) and both ots genes (pWLQ2::otsAB) were constructed and transformed into the C. glutamicum Δ otsAB/Δ treZ mutant. The transformants were checked for their ability to grow in 1 % (w/v) sucrose-containing BMC medium in the absence of trehalose. The plasmid carrying both otsA and otsB efficiently complemented the mutant' s growth deficiency under these conditions. This observation excludes the possibility that the mutant' s growth phenotype is a result of polar effects that could have been caused by the deletion introduced into the chromosome, and also shows that ORF Cgl2574, the ORF located between otsA and otsB on the chromosome which was not supplied on the plasmid, is not essential for trehalose production and normal growth in minimal media. Transformation of the Δ otsAB/Δ treZ double mutant with pWLQ2::otsA led to a significant improvement of growth in 1 % (w/v) sucrose BMC broth, but did not result in the complete complementation of the mutant' s growth deficiency. An explanation for this could be the in vivo substitution of the function of trehalose phosphate phosphatase (OtsB) by a different, perhaps non-specific, phosphatase, or the assumption that the presence of trehalose 6-phosphate instead of trehalose in the C. glutamicum cell is sufficient for a partial restoration of bacterial growth.

### Lipid composition of the trehalose non-producing mutant C. glutamicum Δ otsAB/Δ treZ

As shown here, C. glutamicum mutants impaired in their ability to produce trehalose display significantly impaired growth on minimal media, and this growth deficiency can be complemented by the addition of trehalose to the media. A possible explanation for the importance of trehalose for C. glutamicum growth could be its structural role in the cell. Trehalose is found in C. glutamicum cells not only in its free form but also as mono- and di-esters of the corynomycolic acids which play an important role for the outer cell wall permeability barrier in coryneform bacteria (Puech et al. 2001). It has been shown that trehalose mono- (TMCM) and di- (TDCM) corynomycolates are the dominant components in the non-covalently bound corynomycolate-containing lipid fraction of C. glutamicum (Puech et al. 2000). Our results now demonstrate that the inability of C. glutamicum to synthesize trehalose has a significant influence on the composition of its cell wall lipid fraction.

The Δ otsAB/Δ treZ mutant was grown in 30 ml 1 % (w/v) sucrose-containing BMC broth with or without the addition of 2 % (w/v) trehalose. The cells were harvested after 10 hours of growth and equal amounts of wet cells were used for cell wall lipid isolation as described in Materials and Methods. The lipid fractions of the mutant cells from the trehalose-supplemented and the trehalose-less cultures were characterized and compared with the lipids isolated from the type strain grown under the same conditions. The lipids were separated using silica gel TLC plates developed with a chloroform/methanol/water solvent system. The spots detected after anthrone staining were identified based on the C. glutamicum glycolipid profile described by Puech et al. (2000). When grown in the absence of trehalose, the mutant strain lacked both major trehalose-containing glycolipids in its cell wall lipid fraction. The missing trehalosecorynomycolates were not substituted by other, trehalose-less corynomycolates (such as glucose monocorynomycolate, GMCM, which was observed to be accumulated in a csp1-inactivated C. glutamicum mutant; Puech et al., 2000). In the presence of trehalose in the culture broth, the Δ otsAB/Δ treZ mutant is able to produce trehalose corynomycolates. However, in contrast to the wild-type strain, the trehalose-supplemented mutant contains TMCM as the predominant glycolipid while TDCM was missing. Possibly, the high concentration of trehalose present in the medium results in a shift of the equilibrium in the TDCM synthesis reaction in favor of TMCM (Schimakata & Minatogawa, 2000).

### Construction and characterization of a glgA mutant

C. glutamicum is able to accumulate glycogen in the presence of excess sucrose in the culture medium. In accordance with this observation, a cluster of open reading frames were found in the C. glutamicum genome (Cgl1073-Cgl1072) whose predicted translation products display high-level similarity with enzymes or predicted enzymes of glycogen biosynthesis from some bacteria (Table 3). We decided to disrupt the ORF Cgl1072 which encodes a putative glucosyl transferase which was suspected to represent glycogen synthase (glgA), with two goals in mind: (i) to investigate whether the gene cluster containing this gene is indeed involved in glycogen production by C. glutamicum, and (ii) to find out if glycogen synthesis plays a role in trehalose production.

A mutant designated as glgA::Km was obtained after site-specific integration of pCLiK6::glgA' into the chromosome of C. glutamicum resulting in disruption of the Cgl1072 ORF. The mutant was unable to accumulate glycogen under conditions of excess sucrose. Two additional mutants were made by disruption of the Cgl1072 ORF in the chromosome of the Δ otsAB and Δ otsAB/Δ treS mutants. The mutants were designated as Δ otsAB/glgA::Km and Δ otsAB/Δ treS/glgA::Km, respectively. The phenotypical comparison of the C. glutamicum Δ otsAB/Δ treZ and Δ otsAB/Δ treZ/Δ treS mutants with the two isogenic mutants additionally lacking glycogen synthase (GlgA) did not reveal differences between the four mutant strains with respect to their ability to grow in minimal media without trehalose and their ability to produce and accumulate trehalose. The fact that the glgA::Km and Δ treZ mutants showed identical phenotypes in the Δ otsAB as well as the Δ otsAB/Δ treS background, strongly supports the idea that TreZ and GlgA are involved in one and the same pathway for trehalose biosynthesis. Also, these results provide evidence for the importance of trehalose synthesis from glycogen in C. glutamicum.

### Discussion

### Genetic dissection of trehalose and glycogen biosynthesis pathways in C. glutamicum, and their operation under various growth conditions

Chromosomal mutagenesis was used for inactivation of each of the three trehalose synthesis pathways proposed to exist in C. glutamicum on the basis of the analysis of the available genome sequence data by introducing deletions into selected genes of the pathways. Some of the mutants with a single pathway knocked out showed a decrease in trehalose synthesis but none of them displayed a total lack of trehalose production, suggesting that synthesis of this disaccharide in C. glutamicum is not accomplished by a single pathway, but is based on two or more, presumably coordinately regulated pathways. The subsequent construction of double mutants, in which only one of the three proposed pathways for trehalose synthesis was still active, showed that either the OtsA-OtsB pathway or the TreY-TreZ pathway alone was sufficient to ensure trehalose synthesis at a level meeting the requirements of the bacteria. Even trehalose excretion to the outside of the cells was not dramatically decreased as long as the mutated bacteria possessed one of these two biosynthesis pathways. On the other hand, the inactivation of both the OtsA-OtsB and TreY-TreZ pathways led to the inability of the corresponding mutant to synthesize trehalose and to grow efficiently under most conditions tested. The same result was obtained with a triple mutant where all three trehalose synthesis pathways were inactivated. Thus the pathway inactivation experiments indicate the dominant role of the two pathways involving OtsA-OtsB and TreY-TreZ for the in vivo trehalose synthesis in C. glutamicum.

It is not known if the OtsA-OtsB and TreY-TreZ pathways are used simultaneously in wild-type cells and, if so, if the quantitative contribution of both pathways to trehalose production is similar. From the energetic point of view, the OtsA-OtsB pathway is more efficient than the TreY-TreZ pathway. The sythesis of 1 mol trehalose via the OtsA-OtsB pathway is achieved from 1 mol glucose 6-phosphate and 1 mol UDP-glucose, while 1 mol trehalose produced via the TreY-TreZ pathway consumes 2 moles ADP-glucose (for glycogen synthesis). If one assumes that trehalose is produced mainly for synthesis of the cell wall lipids TDCM and TMCM, and that trehalose phosphate and not free trehalose is needed as a precursor for this purpose (also see below; Shikimakata & Minatogawa, 2000), the energy balance is even more in favor of the OtsA-OtsB pathway, because phosphorylated trehalose is an intermediate of the OtsA-OtsB but not of the TreY-TreZ pathway. Therefore it seems reasonable to speculate that only under energy- and substrate-excess conditions the TreY-TreZ pathway could be preferred over the OtsA-OtsB pathway. On the other hand, our results show that glycogen which can serve as a substrate for the TreY-TreZ pathway is present in C. glutamicum cells also under conditions of low sugar supply, although not in the same amounts as under sugar excess conditions. Also, we observed that the TreY-TreZ pathway alone is sufficient to support C. glutamicum growth not only under sugar excess but also under low-sugar conditions (0.5 % (w/v) sucrose; data not shown). Further experiments are needed to determine the individual contribution of each of the OtsA-OtsB and TreY-TreZ pathways to trehalose biosynthesis in wild-type C. glutamicum cells und different growth conditions.

Our data suggest that the TreS pathway plays only a supporting role in trehalose synthesis. Analysis of the growth and trehalose accumulation characteristics of the Δ otsAB/Δ treZ and Δ otsAB/Δ treZ/Δ treS mutants demonstrated that this pathway is involved in trehalose synthesis during growth on maltose-containing media. It is interesting to note that while the wild-type strain and the Δ otsAB/Δ treZ mutant revealed similar levels of intracellular trehalose, the Δ otsAB/Δ treZ mutant accumulated much less extracellular trehalose than the wild-type whose extracellular trehalose level after growth on maltose was about the same as on sucrose. At present it is not known if the wild-type strain which contains all three functional trehalose biosynthesis pathways preferentially utilizes the TreS pathway during growth on maltose. However, the difference in extracellular trehalose accumulation between the wild-type strain and the mutant retaining the TreS pathway as the only trehalose biosynthesis pathway after growth on maltose suggests that in the wild-type both other pathways have a dominant role for trehalose synthesis also when the bacteria are grown on an excess of maltose.

Our experiments show that the type strain of C. glutamicum accumulates significant amounts of glycogen when grown unders conditions of sugar excess. The genome data predicts the presence in C.glutamicum of a glycogen synthesis pathway using ADP-glucose as precursor, similar to that observed in other bacteria (Preiss & Greenberg, 1965). Using chromosomal insertion mutagenesis, we showed that the ORF Cgl1072 (together with its neighbor Cgl1073) is responsible for glycogen synthesis in C. glutamicum. We were also able to connect glycogen synthesis with trehalose synthesis, showing that otsAB mutants simultaneously impaired in glycogen synthesis (Δ otsAB/glgA::Km and Δ otsAB/Δ treS/glgA::Km) displayed an identical growth and trehalose synthesis phenotype as the otsAB mutants with an inactivated TreY-TreZ trehalose biosynthesis pathway (Δ otsAB/Δ treZ and Δ otsAB/Δ treZ/Δ treS) The growth deficiency of the mutant blocked simultaneously in glycogen synthesis and in the OtsA-OtsB pathway was observed under most growth conditions, including low (1 %) sucrose, which confirms the important role of trehalose biosynthesis from glycogen not only under sugar-excess growth conditions.

### Impact of trehalose biosynthesis on the growth physiology and cell wall lipid composition of C. glutamicum

Revealing the trehalose synthesis mechanisms alone did not give us a direct indication for its physiological role in C. glutamicum. Both the Δ otsAB/Δ treZ and the Δ otsAB/Δ treZ/Δ treS mutants showed a strong trehalose dependence for their growth on the majority of the substrates tested. This result, together with the fact that C. glutamicum and the related mycobacteria (De Smet et al., 2000) have established three independent pathways for trehalose biosynthesis during evolution, indicates the importance of this disaccharide for these bacteria. One of the possible roles of trehalose in C. glutamicum cells is to act as a compatible solute protecting the cells during osmotic shock, a function proposed for trehalose in other bacteria (Argüelles et al., 2000). This hypothesis is supported by the observation of the accumulation of free trehalose in C. glutamicum and Brevibacterium lactofermentum cells under hyperosmotic conditions (Skjerdal et al., 1996). Initial experiments which were carried out to analyse the intracellular and extracellular accumulation of free trehalose in response to changes in the osmolarity of the media were not successful when NaCl was used to adjust the medium' s osmolarity (own unpublished results). A significant increase of the free trehalose levels was obtained only in the presence of high sugar concentrations in the growth media, a finding that correlates with the observation that significantly higher amounts of trehalose were accumulated by the type strain when hyperosmotic stress was induced by sucrose rather than NaCl or glutamate (Skjerdal et al., 1996). In order to further specify the role of trehalose we used the mutants Δ otsAB/Δ treZ and Δ otsAB/Δ treZ/Δ treS which are defective in its synthesis. Both mutants were unable to grow efficienty in minimal media in the absence of trehalose on most of the checked carbon sources. Only the addition of trehalose, but not of other compatible solutes, restored the growth of the mutants. All these results argue against the possibility that trehalose may be synthesized and accumulated in C. glutamicum as a compatible solute in response to changes in osmolarity. Both the intracellular and extracellular trehalose accumulation was shown to be connected with an excess of carbon source in the media and was observed in the late logarithmic and stationary phase. All these prerequisites for trehalose synthesis are reminiscent of conditions known to favor the accumulation of carbon and energy storage compounds such as glycogen in other bacteria. The possibility that trehalose itself is stored as a reserve compound in C. glutamicum, as observed in some higher organisms, is unlikely since the intracellular trehalose level is extremely low in stationary phase cells. The possibility that trehalose accumulation is only a direct result of the glycogen increase in the corynebacterial cells disagrees with the fact that mutants impaired in their ability to synthesize trehalose from glycogen (Δ treZ,Δ treZ/Δ treS) still accumulate significant amounts of trehalose both intracellularly and extracellularly.

The C. glutamicum mutants Δ otsAB/Δ treZ and Δ otsAB/Δ treZ/Δ treS are unable to grow properly under a variety of conditions, and only the addition of trehalose restored growth. These mutants' tendency to form large cell aggregates indicates that their growth problems may be connected with their cell surface or a defect in a late stage of cell division. This suggests that trehalose plays an important structural role for the cells of C. glutamicum. In both mycobacteria and corynebacteria, together with some other closely related genera, it was shown that trehalose in the form of corynomycolic esters is involved in a second permeability barrier outside of the cytoplasmic membrane (Puech et al. 2001, Sathyamoorthy & Takayama, 1987). The characterization of the C. glutamicum glycolipid fraction of a Δ otsAB/Δ treZ mutant shows that one striking consequence of the inability to synthesize trehalose is the absence of trehalose-containing TMCM and TDCM which are thought to be important constituents of the outer lipid bilayer in C. glutamicum. The growth problems of the trehalose-deficient mutants may be connected with their inability to constitute such a cell wall lipid layer. It has been shown that trehalose is not only essential at the final stage of corynomycolate ester metabolism but also, as trehalose phosphate, plays a key role in the entire process of corynomycolic acid synthesis in C. matruchotii (Shimakata & Minatogawa, 2000), i. e. trehalose 6-phosphate was suggested to serve as an acceptor for the fresh synthesized corynomycolic acid. The resulting TMCM is then a common precursor for the synthesis of all esterified corynomycolates of the cell wall, TDCM, and of free corynomycolic acid (Shimakata & Minatogawa, 2000; Puech et al., 2000). Thus, based on this proposal for mycolate biosynthesis by Shikimakata and Minatogawa (2000), the inability to synthesize trehalose or trehalose 6-phosphate by some of the C. glutamicum mutants constructed here could lead not only to the absence of both trehalose-containing glycolipids but also of all other corynomycolate esters. The mechanism just described, where trehalose is used as a carrier for the corynomycolic acid and then is (partially) liberated outside of the cells, may provide an explanation for the presence of extracellular trehalose.

It is interesting to note that on some substrates such as acetate and pyruvate the trehalose-deficient C. glutamicum mutants were able to grow quite normal, reaching similar final culture densities as the wild-type strain, which stands in contrast to the severely impaired growth on sugar substrates. This phenomenon may be explained with differences in the effects an altered cell wall lipid bilayer could have on the uptake of different substrates. Interestingly, in the case of acetate it has be reported that a 50 % decrease in cell wall linked corynomycolates facilitated acetate uptake (Puech et al., 2000).

Importantly, the results of the genetic and physiological dissection of trehalose biosynthesis in C. glutamicum reported here may be of general relevance for the whole phylogenetic group of mycolic acid-containing coryneform bacteria which contains a number of different genera, including medically and biotechnologically important species (see Liebl, 2001). Additional transcriptional and enzyme activity studies are required to reveal the regulation of the trehalose synthesis pathways. Regulation studies are expected to reveal more information about the physiological role of the free extracellular and intracellular trehalose accumulated in C. glutamicum during growth under sugar excess conditions.

### References

Birnboim, H. C. & Doly, J. (1979) A rapid alkaline extraction procedure for screening recombinant plasmid DNA. Nucleic Acids Res 7, 1513-23.

Brana, A. F., Manzantal, & Hardisson, C. (1982) Characterization of intracellular polysaccharides of Streptomyces . Can J Microbiol 28, 1320-1323

Bullock, W. O., Fernandez, J. M. & Short, J. M. (1987) XL1-Blue: a high efficiency plasmid DNA transforming recA Escherichia coli strain with beta-galactosidase selection. BioTechniques 5, 376-379

Cole, S.T., Brosch, R., Barrell, B. G. & other 39 authors (1998) Deciphering the biology of Mycobacterium tuberculosis from the complete genome sequence. Nature 393, 537-44.

De Virgilio, C., Burckert, N., Bell, W., Jeno, P., Boller, T. & Wiemken, A. (1993) Disruption of TPS2, the gene encoding the 100-kDa subunit of the trehalose-6-phosphate synthase/phosphatase complex in Saccharomyces cerevisiae, causes accumulation of trehalose-6-phosphate and loss of trehalose-6-phosphate phosphatase activity. Eur J Biochem 212, 315-23.

Lewington, J., Greenaway, S. D. & Spillane, B. J. (1987). Rapid small scale preparation of bacterial genomic DNA, suitable for cloning and hybridization analysis. Lett Appl Microbiol 5, 51-53

Liebl, W., Bayerl, A., Schein, B, Stillner, U. & Schleifer, K. H. (1989a) High efficiency electroporation of intact Corynebacterium glutamicum cells. FEMS Microbiol Lett 53, 299-303.

Liebl, W., Klamer, R. & Schleifer, K. H. (1989b) Requierment of chelating compounds for the growth of Corynebacterium glutamicum in synthetic media. Appl Microbiol Biotechnol 32, 205-210

Liebl W., Sinskey A. J., Schleifer K. H. (1992) Expression, secretion, and processing of staphylococcal nuclease by Corynebacterium glutamicum. J Bacteriol 174, 1854-61

Liu, J. & Nikaido, H. (1999) A mutant of Mycobacterium smegmatis defective in the biosynthesis of mycolic acids accumulates meromycolates. Proc Natl Acad Sci U S A. 96, 4011-6.

Londesborough, J. & Vuorio, O. E. (1993) Purification of trehalose synthase from baker's yeast. Its temperature-dependent activation by fructose 6-phosphate and inhibition by phosphate. Eur J Biochem. 216, 841-8.

Maruta, K, Kubota, M, Fukuda, S & Kurimoto, M. (2000) Cloning and nucleotide sequence of a gene encoding a glycogen debranching enzyme in the trehalose operon from Arthrobacter sp. Q36. Biochim Biophys Acta 1476, 377-81.

Preiss, J. & Greenberg, E. (1965) Biosynthesis of bacterial glycogen. 3. The adenosine diphosphate-glucose: alpha-4-glucosyl transferase of Escherichia coli B. Biochemistry 4, 2328-34.

Sambrook, J., Fritsch, E. F. & Maniatis, T. (1989). Molecular Cloning: a Laboratory Manual, 2nd edn. Cold Spring Harbor, NY: Cold Spring Harbor Laboratory. Sch5fer, A., Kalinowski, J., Simon, R., Seep-Feldhaus, A. H. & Pühler, A. (1990) Highfrequency conjugal plasmid transfer from gram-negative Escherichia coli to various gram-positive coryneform bacteria. J Bacteriol 172, 1663-6.

Schäfer, A., Tauch, A., Jager, W., Kalinowski, J., Thierbach, G. & Pühler, A. (1994) Small mobilizable multi-purpose cloning vectors derived from the Escherichia coli plasmids pK18 and pK19: selection of defined deletions in the chromosome of Corynebacterium glutamicum. Gene 145, 69-73.
Shimakata, T. & Minatogawa, Y. (2000) Essential role of trehalose in the synthesis and subsequent metabolism of corynomycolic acid in Corynebacterium matruchotii. Arch Biochem Biophys 380, 331-8.
Simic, P., Sahm, H. & Eggeling, L. (2001) L-threonine export: use of peptides to identify a new translocator from Corynebacterium glutamicum. J Bacteriol 183, 5317-24. Simon, R., Priefer, U. & Puehler, A. (1983). A broad host range mobilization system for in vivo genetic engineering: transposon mutagenesis in Gram-negative bacteria. Bio/Technology 1, 784-791
Sathyamoorthy, N. & Takayama, K. (1987) Purification and characterization of a novel mycolic acid exchange enzyme from Mycobacterium smegmatis. J Biol Chem 262, 13417-23.

**Table1. List of C. glutamicum strains used.**

| Strain | Description |
|---|---|
| C. glutamicum DSM 20300 | The type strain of C. glutamicum obtained from DSMZ (Braunschweig, Germany), equal to ATCC13032 strain |
| C. glutamicum Δ otsAB | C. glutamicum DSM 20300 with deletion in the otsA and otsB genes (this work) |
| C. glutamicum Δ treZ | C. glutamicum DSM 20300 with deletion in the treZ gene (this work) |
| C. glutamicum Δ treS | C. glutamicum DSM 20300 with deletion in the treS gene (this work) |
| C. glutamicum Δ otsAB/Δ treZ | C. glutamicum DSM 20300 with deletion in the otsA, otsB and treZ genes (This work) |
| C. glutamicum Δ otsAB/Δ treS | C. glutamicum DSM 20300 with deletion in the otsA, otsB and treS genes (this work) |
| C. glutamicum Δ treZ/Δ treS | C. glutamicum DSM 20300 with deletion in the treZ and treS genes (this work) |
| C. glutamicum Δ otsAB/Δ treZ/Δ treS | C. glutamicum DSM 20300 with deletion in the otsA, otsB, treZ and treS genes (this work) |
| C. glutamicum glgA::Km | C. glutamicum DSM 20300 with insertionally inactivated glgA (this work) |
| C. glutamicum glgA::Km/Δ otsAB | C. glutamicum Δ otsAB with insertionally inactivated glgA (this work) |
| C. glutamicum glgA::Km/Δ otsAB/Δ treS | C. glutamicum Δ otsAB/Δ treS with insertionally inactivated glgA (this work) |
| C. glutamicum Δ otsAB/Δ treZ PWLQ2::otsA | C. glutamicum Δ otsAB/Δ treZ, complemented with the expression plasmid pWLQ2 carrying otsA (this work) |
| C. glutamicum Δ otsAB/Δ treZ PWLQ2::otsA | C. glutamicum Δ otsAB/Δ treZ, complemented with the expression plasmid pWLQ2 carrying otsA (this work) |
| C. glutamicum Δ otsAB/Δ treZ PWLQ2::treZ | C. glutamicum Δ otsAB/Δ treZ, complemented with the expression plasmid pWLQ2 carrying treZ (this work) |
| C. glutamicum Δ otsAB/Δ treZ/Δ treS PWLQ2::treS | C. glutamicum Δ otsAB/Δ treZ/Δ treS, complemented with the expression plasmid pWLQ2 carrying treS (this work) |

**Table 2. PCR primers. The regions that are not homologous to the original gene sequences are shown in italic, the regions that are present only in the original sequence but not in the primer are put in brackets. The restriction sites used for cloning of the purposes are underlined.**

| Primer name | Sequence |
|---|---|
| tre351_f | GGG GAT CC A AAA GAC CAC CGC AAA GAA GAC |
| tre351_r | CCT CTA GAG CAG TAA AGC AAG CGG AAG AA |
| otsAB_f | GGG CAT GC(A) GTA TGC GGA AAG CGT GCG ATT G |
| otsAB_r | GGA AGC TTG CCC CAA ATA ACC GCA AAG CCA |
| treZ_f | GGT CTA GAG CGT TGG TGT AGG CAT TAA C |
| treZ_r | GGT CTA GAC GCA AAA GCC TGG TCA GTT G |
| | |
| treS_f | GGT CTA GA T GAG GCG AAA GTG GTG AAA G T |
| treS_r | GGT CTA GAC ATT CGC GGG ACA ACA CAA T |
| glg_f | GGG TCT AGA GTA TCC ACC AGA GGT TTA CG |
| glg_r | GGG TCT AGA TTA AAT CTT CCG CGT CAT CGA AAG |
| otsB_f | GGG GAT CCA AGG TGC CAG GGC TTT AAA G |
| otsB_r | GGG GAT CCG GAA CCA GAA GTG GAA TTG G |
| treZ_f2 | GGG GAT CCC GGG TGA CTT GCA AAA CCT C |
| treZ_r2 | GGG GAT CCG CAA AAG CCT GGT CAG TTG |
| treS_f3 | GGG TCG ACA TGA GGC GAA AGT GGT GAA AG |
| treS_r3 | GGG TCG ACA CAT TCG CGG GAC AAC ACA A |

**Table 3. Similarity of predicted C. glutamicum enzymes to the enzymes known or predicted to be involved in trehalose biosynthesis. A database search was carried out with a BLAST-based comparison program available online at the National Center for Biotechnology Information server (http://www.ncbi.nlm.nih.gov/BLAST/) using PBLAST, the BLOSUM62 matrix with an EXPECT value of 10 and the low complexity filter.**

| C. glutamicum ORF | LENGTH [AA] | NAME | MATCHING SEQ. | ACCES. NO. | LENGTH [AA] | IDENTITY [%] |
|---|---|---|---|---|---|---|
| Cgl 2573 | 485 | OtsA | Mycobacterium tuberculosis H37RV (OtsA) | G70569 | 500 | 52% |
| | | | Arabidopsis thaliana | AAF99834 | 822 | 38% |
| | | | Saccharomyces cerevisiae (TPS1) | S34979 | 495 | 38% |
| | | | Escherichia coli K12 (OtsA) | P31677 | 474 | 29% |
| Cgl 2575 | 256 | OtsB | Escherichia coli K12 (OtsB) | P31678 | 266 | 26% |
| | | | Mycobacterium tuberculosis H37RV (OtsB2) | C70972 | 391 | 28% |
| | | | Arabidopsis thaliana | AAC39370 | 374 | 28% |
| Cgl 2075 | 566 | TreZ | Mycobacterium tuberculosis H37RV (TreZ) | Q10769 | 580 | 48% |
| | | | Arthrobacter sp. Q36 | S65770 | 598 | 46% |
| | | | Rhizobium sp. M-11 | Q53238 | 596 | 46% |
| | | | Brevibacterium helvolum | 052520 | 589 | 43% |
| Cgl 2066 | 811 | TreY | Mycobacterium tuberculosis H37RV (TreY) | H70763 | 765 | 44% |
| | | | Arthrobacter sp. Q36 (TreY) | S65769 | 775 | 39% |
| | | | Rhizobium sp. M-11 (TreY) | JC4696 | 772 | 39% |
| | | | Brevibacterium helvolum (TreY) | AAB95368 | 776 | 37% |
| Cgl 2250 | 617 | TreS | Mycobacterium tuberculosis H37RV (TreS) | G70983 | 601 | 62% |
| | | | Streptomyces coelicolor A3(2) | CAA04607 | 572 | 64% |
| | | | Pimelobacter sp. R48 | P72235 | 573 | 61 % |
| | | | Thermus aquaticus | 006458 | 963 | 51 % |
| Cgl 1072 | 409 | GlgA | Mycobacterium tuberculosis H37RV | B70610 | 387 | 59% |
| | | | Streptomyces coelicolor A3(2) (glgA) | CAB50741 | 387 | 35% |
| | | | Corynebacterium glutamicum | BAB97794 | 418 | 26% |
| Cgl 1073 | 417 | GlgC | Mycobacterium tuberculosis H37RV (glgC) | 005314 | 404 | 61% |
| | | | Streptomyces coelicolor A3(2) (glgc) | P72394 | 399 | 55% |
| | | | Escherichia coli K12 (glgC) | P31678 | 431 | 36% |

**Table 4. Comparison of the growth of the double mutant Δ otsAB/Δ treZ and the triple mutant Δ otsAB/Δ treZ/Δ treS with the type strain. The strains were grown at 30 °C, 150 rpm, in tubes containing 5 ml BMC broth supplemented with different substrates as specified, at a final concentration of 1 % (w/v) (if not noted otherwise).**

| C-source | WT | Δ otsAB/Δ treZ | Δ otsAB/Δ treZ/Δ treS |
|---|---|---|---|
| Glucose | +++ | +/- | +/- |
| Fructose | +++ | + | + |
| Sucrose (1%) | +++ | +/- | +/- |
| Sucrose (10%) | +++ | +/- | +/- |
| Maltose | +++ | +++ | +/- |
| Trehalose (2%) | - | - | - |
| Sucrose + Trehalose (2%) | +++ | ++ | ++ |
| myo-Inositol | +++ | + | + |
| Pyruvate | +++ | ++ | ++ |
| Accetate | ++ | +(+) | +(+) |

## Claims

1. A method for producing an amino acid comprising culturing a microorganism of the genus Corynebacterium or Brevibacterium wherein said microorganism is partially or completely deficient in at least one of the gene loci of the the group which is formed by otsAB, treZ and treS, and subsequent isolation of the amino acid from the culture medium.

2. A method according to claim 1, wherein the microorganism is deficient in the gene loci of otsAB.

3. A method according to claim 2, wherein the microorganism is deficient additionally in the gene loci of glgA.

4. A method according to claim 3, wherein the microorganism is deficient additionally in the gene loci of treS.

5. A method according to claim 2, wherein the microorganism is deficient additionally in the gene loci of treZ.

6. A method according to claim 5, wherein the microorganism is deficient additionally in the gene loci of treS.

7. A method according to any of the previous claims, wherein the amino acid is drawn from the group consisting of lysine, threonine, methionine and glutamate.
